Europäisches Patentamt

European Patent Office

Office européen des brevets

(19)

(11) Publication number: **0 133 288**
**A2**

(12) # EUROPEAN PATENT APPLICATION

(21) Application number: **84108804.0**

(22) Date of filing: **25.07.84**

(51) Int. Cl.⁴: **C 12 Q 1/68**

(30) Priority: **05.08.83 US 520525**

(43) Date of publication of application: **20.02.85**
**Bulletin 85/8**

(84) Designated Contracting States: **CH DE FR GB IT LI NL SE**

(71) Applicant: **MILES LABORATORIES, INC., 1127 Myrtle Street, Elkhart Indiana 46514 (US)**

(72) Inventor: **Grosch, Josephine C., 54113 Rt. 7-DeCamp Blvd., Elkhart, IN 46516 (US)**
Inventor: **Wilson, Gary A., 54401 Susquehanna Road, Elkhart, IN 46516 (US)**

(74) Representative: **Senftl, Hannes, Dr. et al, c/o Bayer AG Konzernverwaltung RP Patentabteilung, D-5090 Leverkusen Bayerwerk (DE)**

(54) **Bacterial detection by nucleic acid hybridization, labeled probes and test kit therefore.**

(57)  A nucleic acid hybridization method for detecting bacteria in a test sample, such as a mammalian body fluid (e.g., for detecting bacteriuria), employing a polynucleotide probe comprising a base sequence of at least a portion of one of the strands of a tuf or fus gene. Resulting hybridization between the probe and single stranded genomic nucleic acids released from bacteria in the sample indicates bacterial presence and can be detected by employing labeled probes or otherwise detecting duplex formation. The method is particularly applicable to the screening of urine samples for the presence of bacteria belonging to the families Enterobacteriaceae, Psuedomonadaceae, and Streptococcaceae and particularly those of the genera Escherichia, Klebsiella, Enterobacter, Proteus, Psuedomonas, and Streptococcus. The method provides a unique, direct, and rapid means for detecting the presence of bacteria without the need for conventional microbiological culturing.

BACTERIAL DETECTION BY NUCLEIC ACID HYBRIDIZATION,
LABELED PROBES AND TEST KIT THEREFORE

## BACKGROUND OF THE INVENTION

### 1. FIELD OF THE INVENTION

This invention concerns analytical methods and test means for detecting the presence of bacteria in a test sample, particularly, in areas related to health care, such as in the analysis of human body fluids, e.g., urine and blood, for the purpose of aiding diagnosis, or in the testing of foods to detect contamination. The present invention particularly concerns the screening of urine samples from human patients for significant bacterial presence (bacteriuria).

### 2. DESCRIPTION OF THE PRIOR ART

The classical method for detecting bacteria in a test sample involves culturing of the sample in order to expand the number of bacterial cells present into observable colony growths which can be enumerated. If desired, the cultures can also be subjected to additional testing in order to determine antimicrobial susceptibility and identification of particular bac-

MS-1308

terial species. Bacteriological culture methods are particularly labor intensive and require highly skilled technicians. Normally, 24 to 48 hours of incubation are required before a positive or negative result can be reliably determined. Other limitations of the culture method include the need to maintain viability of bacteria in the test sample during transport to and processing at the laboratory.

A wide variety of alternative techniques have been investigated and developed in the continuing attempts to devise methods which overcome the numerous drawbacks of the conventional culture approach. Light microscopy is frequently used to detect bacteria in clinical specimens. Usually the specimen is stained to improve the detection limit and help characterize the organisms present. The method is laborious and does not offer, good detection limits. Immunological methods have been successfully developed to detect specific species and genera which have surface antigens which are distinguishable by specific antibody binding. Such an approach is not practical, however, for quantitating bacteria in a test sample which may contain bacteria from a variety of genera which do not share a common surface antigen from such sources as in body fluids, e.g., blood or urine.

Other tests have been developed based on detection of metabolic products of bacteria such as nitrites, nucleotides such as adenosine triphosphate, $^{14}CO_2$ released from $^{14}C$-labeled glucose, and specific enzymes. The disadvantages of these techniques are many and include: ATP can be degraded by enzymes in the test sample, ATP can originate from nonbacterial sources such as tissues of the host, radioactive materials present on biohazard, and enzymes with similar activity can arise from the host.

Particle counting instrumentation has also been applied to detection of bacteria. Such instrumentation measures perturbations in an electrical current

MS-1308

across a small orifice caused by the presence of particles in a fluid flowing through the orifice. Besides requiring the use of complex and expensive apparatus, this method is highly nonspecific and requires a high level of care and particle-free conditions.

Other types of instrumentation measure the growth of bacteria in special liquid media. The instruments involved make periodic turbidimetric measurements with increases in turbidity indicating the presence of growing bacteria.

Therefore, there is a continuing, long-felt need for a rapid, accurate, and economical technique and means for quantitating bacterial presence. Despite the rapid advance of analytical techniques in closely related fields, as represented by the developments of the radioimmunoassay, spectrophotometry, fluorometry, microcalorimetry, and electrochemical techniques, the predominant technique used in bacteriological testing remains the plate culture method, which today is still much the same as the techniques used by Pasteur and the other early microbiologists of the 19th century. A recent analysis of the state-of-the-art in microbiology concluded that the needs of modern medicine could best be served by developing alternatives to culturing for detecting microorganisms /Nature 302(1983) p. XXVII/.

In 1964, Nygaard and Hall /J. Mol. Biol. 9:125-142 (1964)/ described a method for quantitative detection of DNA/RNA hybrids by filtration of samples through nitrocellulose membranes. They discovered that single-stranded DNA adsorbs onto the membrane, whereas double-stranded (duplexed) DNA and single- or double-stranded RNAs pass through. Sample DNA in single-stranded form and radiolabeled RNA were allowed to incubate in solution resulting in hybridization binding between the radiolabeled RNA and complementary DNA strands in the sample. The reaction mixture was then passed through the nitrocellulose filter. The unhybridized labeled

MS-1308

RNA passed through while the DNA-RNA hybrids had sufficient single-stranded DNA regions to adhere to the filter. Measurement of radioactivity bound to the filter provided an estimate of the amount of hybrid formed.

The birth of this nuclei acid hybridization technique was followed by further refinements and applications to specific analytical situations. Gillespie and Spiegelman /J. Mol. Biol. 12:829-843 (1965)7 fixed single-stranded sample DNA onto nitrocellulose filters and then submerged the filters into a solution of radiolabeled RNA to allow hybridization to occur. Excess labeled RNA was removed by treatment with RNase and washing. A DNA/DNA hybridization method on membrane filters was developed by Denhard /Biochem. Biophys. Res. Commun. 23:641-646 (1966)7. Single-stranded sample DNA was bound to nitrocellulose and treated with a prehybridization solution to minimize nonspecific binding of labeled probe DNA during the hybridization reaction. Grunstein and Hogness /Proc. Natl. Acad. Sci. USA 72:3961-3965(1975)7 developed a solid phase colony hybridization technique for rapid screening of E. coli colonies to determine which ones contained an inserted DNA sequence. Immobilization of nucleic acids to solid supports has also been improved to include RNA and short DNA fragments. The techniques developed have included using paper with chemically reactive groups /Alwine et al, Proc. Natl. Acad. Sci. USA 74:4350(1977), Seed, Nucleic Acids Res. 10:1979(1982), and Hunger, Bioch. Biophys. Acta 653:344(1981)7 and derivatization of the nucleic acid prior to fixation on nitrocellulose /Thomas, Proc. Natl. Acad. Sci. USA 77:5201(1980)7.

Nucleic acid hybridization assays have been developed for a number of diseases. These include the genetic diseases $\alpha$-thalassemia /Kan et al. New Eng. J. Med. 295:1165(1976)7, ß-thalassemias /Orkin

MS 1308

et al, Nature 296:627(1982)_7 and sickle cell anemia /Kan and Dozy, Proc. Natl. Acad. Sci. USA 75:5631 (1978), Geever et al, ibid 78:5081(1981), Wilson et al, ibid 79:3628(1982) and Conner et al, ibid 80:278 (1983)_7. Nucleic acid hybridization tests for the presence of infectious agents have also been reported. These include a test for enterotoxin-producing bacteria in fecal specimens /Moseley et al, J. Infect. Dis. 42:892(1980), ibid 115:563(1982), and U.S. Pat. No. 4,358,535_7. See also Institut Pasteur, British Pat. 2,019,408B. Hepatitis virus in serum has also been detected by hybridization /Schafritz et al, Proc. Natl. Acad. Sci. USA 79:5675(1982) and Beminger et al, J. Med. Virol. 9:57(1982)_7.

Some studies have been undertaken of the generic relatedness of various bacteria based on nucleic acid hybridization between denatured and fragmented single-stranded DNA from different bacteria /see Brenner, The Public Health Laboratory, Thompson Publ. Inc., vol. 32 (1974) pp. 118-130; and the review by Brenner and Falkow, Adv. in Genetics 16:81-119(1972)_7. Conservation of bacterial genomic DNA coding for various products such as ribosomal RNA, ribosomal proteins, transfer RNAs, and the like has been the subject of some study /Biophys. J. 8:1105-1118(1968); PNAS 76: 381-385(1979); J. Bact. 133:1089(1978); J. Biol. Chem. 255:728-734(1980); and J. Bact. 129:1435(1977)_7. Filer et al /Eur. J. Biochem. 120:79-77(1981)_7 have reported that DNA sequences homologous to certain prokaryotic genes, e.g., E. coli tuf genes, are present in some taxonomically unrelated genera.

MS-1308

- 6 -     0133288

## SUMMARY OF THE INVENTION

It has now been found that bacteria can be detected in a variety of test samples by nucleic acid hybridization employing *tuf* and *fus* gene probes. In particular, the present method can be advantageously applied to the screening of mammalian, e.g., human, body fluids, especially urine samples, for the presence of pathologically significant numbers of bacteria.

The test sample is first subjected to conditions to release and denature (render single stranded) genomic nucleic acids from bacteria present in the sample. The resulting single stranded nucleic acids are then contacted in an appropriate manner with a polynucleotide probe comprising a base sequence of at least a portion of a gene selected from *tuf* and *fus* genes. Contact between the probe and the denatured sample nucleic acids is performed under conditions favorable to hybridization between the probe and homologous base sequences in the genomic nucleic acids of said bacteria. Resulting hybridization is then determined as a function of the extent of bacterial presence in the test sample.

The homologous base sequence of the probe is preferably at least about 10, normally at least about 20, and usually several hundred, bases in length. It is especially preferred to use a probe comprising an *E. coli tuf* gene, or a segment thereof, when applying the present method to the testing of samples wherein, if they contain significant numbers of bacteria, such bacteria would be expected to be predominantly of the *Escherichia* genus.

MS-1308

Detection of hybridized probe can be preferably accomplished by solid phase hybridization involving use of a labeled form of the probe. The label can be any convenient detectable moiety such as radioactive isotopes or nonradioactive elements such as fluorescers, chemiluminescers, or molecules which form specific binding sites (e.g., haptens) for binding partners (e.g., antibodies) incorporated with a directly detectable species (e.g., enzymes, enzyme substrates, cofactors, or modulators, fluorescers, and chemiluminescers).

The present method for detecting bacteria has numerous advantages over the prior art techniques, particularly the classical culturing methods. In the present method it is not necessary to isolate or propagate the bacteria to be detected, which in some cases can be a highly infectious agent. Furthermore, it is not necessary to rely on the expression of certain metabolic products or surface antigens in order to make a determination. In addition, the present method does not require that test samples be handled in a manner necessary to guarantee viability of any bacteria present. The prior art methods in general will detect only viable cells in the sample. Often, microbes die during transport of specimens to the laboratory for testing. Also, a specimen can contain different types of microorganisms having a variety of growth limitations and requirements. Thus, when using the prior art methods, the specimen must be cultured under several different conditions in order to ensure detection of any possible bacteria present. Such a serious limitation is absolutely not required by the present method since the object of the assay is the stable nucleic acid composition of the bacteria present in a sample.

MS-1308

DESCRIPTION OF THE PREFERRED EMBODIMENTS

The use of nucleic acid hybridization as an analytical tool is based fundamentally on the double-stranded, duplex structure of DNA. The hydrogen bonds between the purine and pyrimidine bases of the respective strands in double-stranded DNA can be reversibly broken. The two complementary single strands of DNA resulting from this "melting" or "denaturation" of DNA will associate (sometimes referred to as reannealing or hybridization) reforming the duplexed structure. As is now well known in the art, contact of a first single stranded nucleic acid, either DNA or RNA, which comprises a base sequence sufficiently complementary to (i.e., "homologous with") a second single stranded nucleic acid under appropriate solution conditions, will result in the formation of DNA/DNA, RNA/DNA, or RNA/RNA hybrids, as the case may be.

In accordance with the present invention, a polynucleotide (normally single stranded DNA or RNA) is selected as an analytical reagent on the basis that it comprises a base sequence which is homologous, i.e., sufficiently complementary, with base sequences found in substantially all bacteria of interest. Thus, hybridization between this polynucleotide probe and nucleic acids released in single stranded form from the test sample indicates the presence of bacteria in the sample.

The present invention is principally founded on the selection of the *tuf* and *fus* genes in bacteria, especially *E. coli*, as the source of the hybridization probe. The *tuf* and *fus* genes code for the synthesis of EF-Tu and EF-G elongation factors, respectively, which are involved in the translational process of protein synthesis in the bacterial cell. Elongation factors in general mediate the entry of an aminoacyl-tRNA into the A site of the ribosome. The EF-Tu protein carries a guanine nucleotide (GTP) and the complex binds aminoacyl-tRNA. EF-Tu is understood to be composed of a single polypeptide chain of 393 amino acids having a molecular weight of about 43,000. In *E. coli*, there are two genes, *tuf A* and *tuf B*, which code for EF-Tu. There are about 70,000 molecules of EF-Tu per cell on average, or 5% of the total protein, making this a major protein in *E. coli*.

The *tuf* gene is understood to comprise about 1200 base pairs. In *E. coli*, the *str* operon contains both the *fus* gene for EF-G and the *tuf A* gene for EF-Tu, as well as genes for ribosomal proteins S7 and S12. It is preferred to use an approximately 800 base pair (bp) fragment of *E. coli tuf A* gene delineated by Kpn (K) and Eco R1(E) restriction enzyme sites. Useful techniques for cloning *tuf* and *fus* gene fragments are described by Jaskunas *et al, Nature 257*:458-462(1975). Once the desired probe sequence has been isolated or, as will be more usual, has been cloned in an appropriate vector such as pBR322, pHV33, pUB110, Lambda and derivatives thereof, and pYIP12, it can be used directly in hybridization assays. A particularly preferred vector is M13 bacteriophage since only single stranded DNA probe will be generated, thereby eliminating the need to denature the probe prior to use in the assay and preventing self-hybridization [*Molecular Cloning, A Laboratory Manual*, T.Maniatis et al, Cold Spring Harbor Laboratory(1982) pp. 51 *et seq.*].

MS-1308

The extent and specificity of nucleic acid reassociation is affected by:

1.   The purity of the nucleic acid preparation.

2.   Base composition of the probe - G-C base pairs will exhibit greater thermal stability than A-T base pairs.  Thus, hybridizations involving higher G-C content will be stable at higher temperatures.

3.   Length of homologous base sequence - Any short sequence of bases (e.g., less than 6 bases), has a high degree of probability of being present in many nucleic acids.  Thus, little or no specificity can be attained in hybridizations involving such short sequences.  The present homologous probe sequence will be at least 10 bases, usually 20 bases or more, and preferably greater than 100 bases.  From a practical standpoint, the homologous probe sequence will often be between 300-1000 nucleotides.

4.   Ionic strength - The rate of reannealing increases as the ionic strength of the incubation solution increases.  Thermal stability of hybrids also increases.

5.   Incubation temperature - Optimal reannealing occurs at a temperature about 25-30°C below the melting temperature (Tm) for a given duplex.  Incubation at temperatures significantly below the optimum allows less related base sequences to hybridize.

6.   Nucleic acid concentration and incubation time - Normally, to drive the reaction towards hybridization, one of the hybridizable sample nucleic acid or probe nucleic acid will be present in excess, usually 100 fold excess or greater.

7. Denaturing reagents - The presence of hydrogen bond disrupting agents such as formamide and urea increases the stringency of hybridization.

8. Incubation time - The longer the incubation time the more complete will be the hybridization. Normally, incubation times are between 6 and 24 hours.

9. Volume exclusion agents - The presence of these agents, as exemplified by dextran and dextran sulfate, are thought to effectively increase the concentration of the hybridizing elements thereby increasing the rate of resulting hybridization.

Based on the above criteria and related factors known in the art, contact between the probe and denatured sample nucleic acids will be accomplished under conditions favorable to hybridization. As conditions are made more stringent, greater complementarity is required for formation of probe hybrids. Hybridization conditions of low salt and high temperature increase the stringency of annealing. One of the most popular conditions for hybridization is in 2XSSC (where 1XSSC = 0.15M sodium chloride and 0.015M sodium citrate, pH 7.0) at 65°C. Under such conditions, hybridization generally will require at least about 15-20 contiguous, exactly matched base pairs in the probe.

In some applications, it may be desirable to allow hybridization between less complementary sequences, e.g., to allow for evolutionary mutations affecting nucleic acid sequence but not overall cellular function and structure. The ability of related but not identical complementary sequences to reanneal can be controlled by the conditions of stringency. High stringency, e.g., elevated temperatures, requires formation of greater numbers of base pairs in a given length of duplex since reassociation is restricted to closely related sequences. At lower temperatures, i.e., less stringency, more distantly related sequences can reanneal. Additionally, increasing incubation time also permits less related sequences to anneal.

Practice of the present analytical method is not limited to any particular hybridization format. The manner in which hybridization resulting between the probe and sample nucleic acids is determined is primarily a matter of convenience. Any conventional hybridization technique can be used. As improvements are made and as conceptually new formats are developed, such can be readily applied to carrying out the present method.

Conventional hybridization formats which are particularly useful include those wherein the sample nucleic acids or the polynucleotide probe is immobilized on a solid support (solid-phase hybridization) and those wherein the polynucleotide species are all in solution (solution hybridization).

A. Solid-phase Hybridization

In this approach, one of the polynucleotide species participating in hybridization is fixed in an appropriate manner in its single stranded form to a solid support. Useful solid supports are well known in the art and include those which bind nucleic acids either covalently or noncovalently. Noncovalent supports which are generally understood to involve hydrophobic bonding include naturally occurring and synthetic polymeric materials, such as nitrocellulose, derivatized nylon, and fluorinated polyhydrocarbons, in a variety of forms such as filters or solid sheets.

MS-1308

Covalent binding supports are also useful and comprise materials having chemically reactive groups or groups, such as dichlorotriazine, diazobenzyloxymethyl, and the like, which can be activated for binding to polynucleotides.

A typical solid-phase hybridization technique begins with immobilization of sample nucleic acids onto the support in single stranded form. This initial step essentially prevents reannealing of complementary strands from the sample and can be used as a means for concentrating sample material on the support for enhanced detectability. The polynucleotide probe is then contacted, in a single stranded, labeled form, with the support. Appropriate labels are available by which to detect resulting hybridization on the support. Typically, a solid-phase hybridization technique will proceed as follows:

(1) the test sample is subjected to conditions to release and denature bacterial nucleic acids and resulting single stranded nucleic acids are immobilized on a solid support, e.g., a liquid sample such as a body fluid is applied to a nitrocellulose membrane, the deposited cells are lysed and released DNA is denatured, and the membrane is baked *in vacuo* at 80°C for 2 hours to fix single stranded DNA to the membrane; alternatively, the cells are first lysed and released DNA denatured and then applied to the nitrocellulose membrane;

(2) the support is contacted with the labeled probe in excess under favorable hybridization conditions, e.g., after saturating all nonspecific DNA binding sites on the membrane by treatment at 40-60°C with a prehybridization solution comprising buffer (e.g., 2XSSC), protein such as bovine serum albumin, Ficoll (a trademark identifying a copolymer of sucrose and epichlorohydrin sold by Pharmacia Fine Chemicals,

MS-1308

Piscataway, NJ), polyvinylpyrrolidone, and a denatured foreign DNA such as from calf thymus or salmon sperm; typically the hybridization conditions will be the same as the prehybridization condition except the time of incubation will usually be longer;

(3)   removing labeled probe which has not become associated with the support by hybridization, e.g., by simple washing of the membrane; and

(4)   measuring the label on the support in accordance with the detectable characteristic of the label.

Traditionally, the label will comprise a radioisotope such as $^{32}P$ and will be detected by scintillation counting or autoradiography, however, as will be more fully described below, nonradioisotopic detection schemes can also be used.

Additional steps may also be included in the above typical protocol.  For example, where particularly short DNA fragments (e.g., less than about 1000 bases) or RNAs are to be immobilized, such polynucleotides can be first derivatized with glyoxal and then applied to the support.  Alternatively, reactive cellulose can be used to covalently bind the polynucleotides, usually after an initial purification of the sample to isolate nucleic acids according to standard methods.

An example of a hybridization format using covalent binding, chemically derivatized paper, such as diazobenzyloxymethyl cellulose, is as follows:

(1)   release and purify nucleic acids from bacteria present in the sample,

(2)   denature the sample nucleic acids by heating at 100°C for several minutes,

(3)   spot the denatured mixture onto the chemically activated paper,

(4)   allow to air dry, then store for 12-16 hours in a closed container,

(5)   wash the paper with water, 0.4N sodium hydroxide, and again with water,

MS-1308

(6) rinse the paper with prehybridization buffer,

(7) incubate the paper for 1 hour in an elution buffer [e.g., 99% (v/v) deionized formamide in 10 mM Tris. HCl buffer (pH 7.8)],

(8) wash the paper again with prehybridization solution,

(9) add labeled probe in hybridization solution,

(10) wash the paper to remove excess, unhybridized probe, and

(11) measure the label on the paper.

Instead of immobilizing sample nucleic acids and contact with labeled probe, it is also possible to label sample nucleic acids *in situ* by known methods and thereafter add the probe in immobilized form. The end measurement is the same, detection of the label associated with the support.

Another method of interest is the sandwich hybridization technique wherein one of two mutually exclusive fragments of the homologous sequence of the probe is immobilized and the other is labeled. The presence of bacterial nucleic acids results in dual hybridization to the immobilized and labeled probe segments, again with the same ultimate measurement of support-associated label. See *Methods in Enzymology* 65:468(1980) and *Gene* 21:77-85(1983) for further details.

MS-1308

An additional assay method would use a DNA probe which is complementary to a conserved sequence of an RNA such as 5S or 16S RNA. The probe would be hybridized with the RNA from the sample and the RNA/DNA duplex would be detected in any convenient manner, such as with labeled antibody specific for RNA/DNA hybrids [see Rudkin and Stollar, *Nature 265*:472 & 473 (1977), Stuart *et al*, *PNAS 78*:3751(1981), Reddy and Sofer, *BBRC 103*:959-967(1981), and Nakazato, *Biochem. 19*: 2835-2840(1980)]. This method offers improved detection because hundreds or thousands of copies of the complementary RNA can occur per cell.

B. Solution Hybridization

The present method can also be used for detection of bacterial nucleic acids in a solution format. This normally requires that the homologous sequence be in single stranded form, be it RNA or DNA. This will be referred to as the probe polynucleotide.

MS-1308

In a solution format, the specimen nucleic acids are first released from bacterial cells in the sample by lysis, and then denatured. These steps may be combined by heating the sample to 100°C or by exposing it to base. After adding a solution containing a large excess of the probe, hybridization is allowed to occur under conditions of ionic strength and temperature empirically determined to give the desired probe specificity and sensitivity.

Hybrids can be detected and quantitated using a number of methods. For example, after hybridization the remaining single stranded nucleic acid can be hydrolyzed into small fragments with the single-strand specific nuclease $S_1$. Acid precipitation followed by centrifugation or filtration can be used to concentrate the hybrids and separate them from the hydrolyzed single-stranded polynucleotides. The amount of precipitate collected is then quantitated. In another approach, hybridized and single-stranded polynucleotides can be separated by chromatography on hydroxyapatite. Other solution methods are known and will be developed.

C.    Labels

Preferably, when used as a labeled reagent, the probe polynucleotide is single stranded DNA or RNA thereby obviating the need for denaturation of the probe prior to hybridization. This can be done by inserting the probe sequence into a single-stranded DNA bacteriophage (e.g., M13) or by isolating a labeled probe after an *in vitro* polymerization reaction. The probe or other material to be labeled can be incorporated with a variety of labels. Useful labels include radioisotopes as well as nonradioisotopic labels. Isotopic labels include, but are not limited to, $^3H$, $^{35}S$, $^{32}P$, $^{125}I$, and $^{14}C$. The method of radioactively labeling the probe or other material will depend upon its particular nature (e.g., RNA vs. DNA, single-stranded vs. double-stranded). Most labeling methods are enzymatic. These include, but are not limited to, the known methods of nick translation, end labeling, second strand synthesis, reverse transcription, and transcription. All of these methods generally require the isotopically labeled nucleotides to function as enzyme substrates.

Alternatively, a radiolabel can be incorporated into the polynucleotide probe by chemical modification of the polynucleotide probe. This method is used most commonly with $^{125}I$ labels.

With a radiolabeled polynucleotide probe, hybridization can be detected by autoradiography., scintillation counting or gamma-counting. The method used would depend upon the hybridization format, the type of test (qualitative or quantitative), and the radioisotope used as a label.

Nonradioisotopic materials can also be used as labels. Such labels can be incorporated into the polynucleotide probe or other material to be labeled by enzymatically incorporating modified nucleotides using one of the enzymatic procedures outlined above where the labeled nucleotides serve as enzyme substrates for the appropriate enzymes. Alternatively, a label could be introduced into a polynucleotide probe by conventional chemical modifications of the probe.

Useful labels include, but are not limited to, haptens or other ligands, fluorescers, chemiluminescers, chromophores, and participants in enzymic reactions (e.g., enzymes, enzyme cofactors, enzyme substrates, and enzyme modulators, e.g., inhibitors). These labels

MS-1308

are detected on the basis of their own physical properties (e.g., fluorescers and chromophores) or their reactive properties (e.g., the others listed). For example, a cofactor-labeled probe can be detected by adding the enzyme for which the label is a cofactor and a substrate for the enzyme. A hapten and ligand labeled polynucleotide probe can be detected by adding an antibody to the hapten or a protein which binds the ligand, tagged with a detectable molecule. Such detectable molecule can be some molecule with a measurable physical property (e.g., fluorescence or absorbance) or a participant in an enzyme reaction (e.g., see above list). For example, one can use an enzyme which acts upon a substrate to generate a product with a measurable physical property. Examples of the latter include, but are not limited to, β-galactosidase, alkaline phosphatase and peroxidase. For *in situ* hybridization studies, ideally the final product is water insoluble.

A particularly useful labeling approach is a variation of second strand synthesis which can be applied to a single stranded DNA vector containing a region of defined sequence on the 5' side of the probe sequence [Hu and Messing, *Gene 17*:271-277(1982)]. In this approach, an oligonucleotide primer complementary to the sequence on the 5' side of the probe is used to initiate second strand synthesis with DNA polymerase. An appropriate label (which may be radioactive or nonradioactive as described above) can be incorporated into the second DNA strand. This approach gives a polynucleotide which is single stranded in the probe region, but which will contain a multiply labeled, double stranded region on the 5' side of the probe sequence.

A technique not requiring incorporation of a label into a polynucleotide probe has been described by Rudkin and Stollar [*Nature* 265:472-473(1977)] and used by cytochemists for detection of hybridization *in situ*. In this approach, single stranded RNA containing the desired sequence is hybridized to DNA from the sample. Hybridization is detected using labeled antibodies specific for RNA/DNA hybrids (these antibodies do not bind to double stranded DNA or single stranded RNA or DNA). This approach is applicable to the present method. Preferred antibody labels would be those described above.

In general, labels and labeling approaches which have been developed for use in immunoassays will be applicable to the present hybridization technique with modifications evident to the ordinary skilled worker. See U.S. Pat. Nos. 4,380,580; 4,279,992; 4,238,565; 4,134,792; 4,273,866; 3,817,837; 4,043,872; 4,238,195; 3,935,074; 3,998,943; 3,654,090; 3,992,631; 4,160,016; and 3,996,345; British Pat. Specs. 1,552,607 and 3,019,408; and European Pat. Applns. 70,687; 70,685; and 63,879; the pertinent parts of which relating to labeling schemes are incorporated herein by reference.

It will be clearly recognized that the present invention is not limited to any particular hybridization format or formats or particular labels. As new techniques and labels are developed, they will be applicable to the present method. Reference to *Molecular Cloning, A Laboratory Manual (supra)* will provide additional details concerning nucleic acid hybridization.

The test sample to be assayed for bacterial presence can be any medium of interest, and will usually be a liquid sample of medical, veterinary, environmental, nutritional, or industrial significance. Human and animal specimens and body fluids particularly can be assayed by the present method, including urine, blood (serum or plasma), milk, cerebrospinal fluid, sputum, fecal matter, lung aspirates, throat swabs, genital swabs and exudates, rectal swabs, and nasopharnygal aspirates. The present invention has particular advantages for the detection of bacteria in urine (bacteriuria). The detection of $10^5$ or more bacterial colony forming units per milliliter in a urine sample is presumptive of a urinary tract infection. In uncomplicated infections, the etiological agents comprise two general categories according to incidence:

MS-1308

Category 1     (90% incidence)
    Family:  *Enterobacteriaceae* (Gram-negative
      facultatively anaerobic rods)
      *Escherichia (E. coli)*
      *Klebsiella (K. pneumoniae)*
      *Enterobacter (E. cloacae, E. aerogenes)*
      *Proteus (P. vulgaris, P. mirabilis,*
      *Morganella morganii)*
Category 2 (5-10% incidence)
    Family: *Pseudomonadaceae* (Gram-negative
      aerobic rods and cocci)
      *Pseudomonas (P. aeruginosa)*
    Family: *Micrococcaceae* (Gram-positive cocci)
      *Staphylococcus (S. aureus)*
    Family: *Streptococcaceae* (Gram-positive cocci)
      *Streptococcus* Group D *(Enterococci)*

In complicated infections, strains of *Citrobacter,*
*Serratia,* and *Providencia* also may appear. Probes
homologous for this entire group or subsets thereof
can be obtained according to the present invention
depending on the degree of specificity and sensitivity
desired for a particular bacteria test.

While the present invention has been particularly
described with reference to, and is most advantageously
applied to, the detection of bacteria, it will be
understood that this method can be used to detect other
types of microorganisms such as protozoa, yeasts,
viruses, and so forth.

The test sample can be treated in a variety of
known manners in order to release bacterial nucleic
acids in single stranded form. Release of nucleic
acids can, for example, be obtained by mechanical disruption (freeze/thaw, abrasion, sonication), physical/
chemical disruption (detergents such as Triton, Tween,
sodium dodecylsulfate, alkali treatment, osmotic

MS-1308

shock, heat-boiling water), or enzymatic lysis (lysozyme, proteinase K, pepsin). Denaturation of released nucleic acids is preferably accomplished by heating in boiling water or alkali treatment (e.g., 0.1 N sodium hydroxide), which, if desired, can simultaneously be used to lyse the cells.

The present invention additionally provides a reagent system, i.e., reagent combination or means, comprising all of the essential elements required to conduct a desired assay method. The reagent system is presented in a commercially packaged form, as a composition or admixture where the compatability of the reagents will allow, in a test device configuration, or as a test kit, i.e., a packaged combination of one or more containers, devices, or the like holding the necessary reagents, and usually including written instructions for the performance of assays. Reagent systems of the present invention include all configurations and compositions for performing the various hybridization formats described herein. Particularly preferred is a test kit for performing a solid-phase hybridization protocol comprising (1) a solid support capable of immobilizing single stranded nucleic acid resulting from treatment of a test sample to release and denature bacterial nucleic acids, and (2) a labeled polynucleotide probe selected from the various types described herein. Preferably, such kit will additionally comprise foreign nucleic acid for use in substantially saturating nucleic acid adsorption sites on the support after immobilization of sample nucleic acid, along with, optionally, other desired ingredients for a prehybridization solution. Also, the kit will preferably include a chemical lysing and denaturing agent, e.g., alkali, for treating the sample to release single stranded nucleic acid therefrom. Ingredients for the hybridization reaction, if different from the prehybridization solution, will also be preferably

MS-1308

- 24 -

included in the kit.  The reagent system can, of course, include other materials and solutions as are known in the art and which may be desirable from a commercial and user standpoint, such as buffers, diluents, standards, and so forth.

The present invention will now be illustrated, but is not intended to be limited, by the following examples.

MS-1308

## EXAMPLE 1

A.  *Growth of microorganisms used as test strains.*

The organism *Escherichia coli* No. 063 (University of Rochester Medical Center, Rochester, NY) was obtained from the urine of a patient that tested positive for urinary tract infection.  As a control non-urinary tract infection isolate, the microorganism *Bacillus subtilis* (No. 1A289 from the Bacillus Genetic Stock Center, Ohio State University, Columbus, OH) was used. Each organism was grown in a liquid nutrient medium in the following manner.

Each shake flask was inoculated with either *E. coli* or *B. subtilis*.  The shake flasks contained fifty milliliter (ml) of 2XYT broth.  2XYT consists of 1.6% Bactotryptone, 1.0% yeast extract and 0.5% sodium chloride.  Bactotryptone and yeast extract are available from Difco Laboratories, Detroit, MI.  Both cultures were grown at 37°C in a rotary shaker for 6 hours.

In order to determine the bacterial count per unit volume of each 6 hour broth culture, the culture was serially diluted by ten-fold increments into 2XYT broth. Each of the dilutions were plated on sterile agar medium containing tryptose blood agar base (TBAB) and 0.1% casamino acids (both components available from Difco).  Those plates were incubated at 37°C for 12 hours to allow colonies to form.  The plates containing 30-300 colonies were counted and the number of cells per unit volume in the original broth cultures was determined by calculation.

MS-1308

B.  *Application of cells from broth culture to a solid support for immobilization of nucleic acids.*

The six-hour broth cultures and freshly made dilutions of these cultures were applied to a solid support consisting of nitrocellulose (Bio-Rad Trans-blot$^{TM}$ transfer medium available from Bio-Rad Laboratories, Richmond, CA). The addition of a precise number of cells to a constant surface area of the nitrocellulose was accomplished by the use of an apparatus called a Minifold$^{TM}$ which is commercially available from Schleicher & Schuell, Inc., Keene, NH. This apparatus is a filter manifold consisting of three sections which are clamped together into a "sandwich" during the filtration or immobilization process. The Minifold$^{TM}$ apparatus was fitted with one 4 inch (") x 5 1/4" sheet of the nitrocellulose membrane, backed by two 4" x 5 1/4" sheets of Whatman 3 MM chromatography paper (available from Whatman Ltd., England). The nitrocellulose and Whatman paper were presoaked in five ml of 1XSSC (1XSSC = 0.15M NaCl, 0.015M trisodium citrate). The membrane and backing papers were clamped into the apparatus. The apparatus was hooked up to a vacuum line and the vacuum applied. To each well was added 0.10 ml of the 6 hour *E. Coli* culture and to successive wells, 0.10 ml the serial dilutions of this culture. The control 6 hour culture of *B. subtilis* was also collected on the filter in the same fashion as described for the *E. coli* culture. As an additional control, nutrient media was added to another set of wells.

C.  *Lysis of cells and denaturation of DNA on*
    *membrane containing the cells.*

The nitrocellulose membrane containing the cells was removed from the Minifold<sup>TM</sup> apparatus and placed on a Whatman 3MM paper which had been saturated with a 0.5M sodium hydroxide solution.  The membrane was placed on paper so the cells were always on the upper surface.  The nitrocellulose membrane remained in contact with the 3MM paper for 10 minutes at 65°C. The nitrocellulose membrane was then placed on a Whatman 3MM paper that had been presoaked with 1M Tris, pH 7.4.  The nitrocellulose remained in contact with the paper for 10 minutes at 65°C.  The nitrocellulose membrane was removed and placed on a second 3MM paper saturated with 1M Tris, pH. 7.4, for 10 minutes at 65°C.  The nitrocellulose was removed and placed on a Whatman 3MM paper saturated with a solution of 0.5M Tris, 1.5M NaCl, pH 7.4, for 10 minutes at 65°C.  The nitrocellulose membrane was then air dried and baked at 80°C for 2 hours in a vacuum oven.

In a typical experiment, samples containing $1 \times 10^8$, $1 \times 10^7$, $1 \times 10^6$, $1 \times 10^5$, $1 \times 10^4$, $1 \times 10^3$, $1 \times 10^2$, $1 \times 10^1$ cells were applied to each well.

MS-1308

D. *Isolation and purification of M13-10 replicative form DNA.*

The bacteriophage M13-10 (ATCC 39403-B1) was used as the source of the *tuf* A gene fragment. The *tuf* A gene of *E. coli* encodes for the protein EF-Tu. EF-Tu is a single polypeptide chain with a molecular weight of 43,225. The EF-Tu protein is an elongation factor in protein synthesis and mediates the entry of an aminoacyl-tRNA into the A site of the ribosome. The EF-Tu protein carries a guanine nucleotide (GTP). The EF-Tu-GTP binds aminoacyl-tRNA. The size of the *tuf* A gene is 1,190 base pairs. The portion of the *tuf* A gene contained in M13-10 is an 800 base fragment. The *tuf* A gene is cloned between the Hinc II and EcoRI restriction endonuclease sites of the vector M13mp9 (New England BioLabs, Beverly, MA).

The *Escherichia coli* host organism used for growth of M13-10 phage was JM103 (Δ*lac, pro), supE, thi, strA, endA, sbcB15, hsdR4, traD36, lac IqZM13*. This strain is commercially available from Bethesda Research Laboratories, Gaithersburg, MD.

Fifteen ml of an overnight culture of the above *E. coli* was inoculated into one liter of 2XYT broth in a two liter Fernbach flask. This culture was grown for 3 hours at 37°C on a New Brunswick Gyrotary™ shaker (200 rpm). To the 3 hour culture $2 \times 10^{11}$ PFU (plaque forming units) of M13-10 bacteriophage were added. This infected culture was allowed to incubate for 1 hour under the same conditions as described above. At this time, 150 mg of chloramphenicol was added to the culture. Incubation was continued for an additional 45 minutes. Cells were harvested by centrifugation (GSA rotor, 5,000 rpm, 10 minutes) and washed once in cold 2XYT broth. The washed cells were suspended in 40 ml cold 10% sucrose solution. Lysis was accomplished by the addition of 20 mg of lysozyme and 16 ml of 0.25M ethylenediaminetetraacetic acid (EDTA) followed by incubation in an ice bath for 10 minutes. Lysis was completed by the addition of 4 ml 20% sodium dodecyl sulfate (SDS). The lysis mixture was mixed gently. The concentration of sodium chloride in the lysate solution was adjusted to 1M with the addition of 2.90 gm of sodium chloride. The lysate mixture was incubated at 40°C overnight. The lysate was cleared by 4°C centrifugation in a Beckman ultracentrifuge at 60,000 xg for 30 minutes. The supernatant was extracted three times with equal volumes of a phenolchloroform mixture composed of 4 parts of phenol - 1 part of chloroform. The aqueous phase from the extraction was added to two volumes of cold 95% ethyl alcohol. This mixture was incubated at -70°C for thirty minutes and thencentrifuged 1,800 xg for 15 minutes at 4°C. The pellet that resulted was washed with 70% ethyl alcohol and then air dried. The DNA pellet was dissolved in 15 ml of a solution containing 0.01M Tris, pH 8, 0.001M EDTA. This DNA solution was subjected to isopycnic cesium chloride density gradient centrifugation in the presence of ethidium bromide to separate covalently closed circular replicative form DNA from linear DNA. Cesium chloride was added to the DNA solution at ambient temperature to a final refractive index of 1.3930. After centrifugation for 40 hours at 130,000 xg, the lower band containing the replicative form DNA was removed and extracted 6 times with water-saturated sec-butanol to remove ethidium bromide. The extract was dialyzed extensively against TE buffer (0.01M Tris, pH 8.0, 0.001M EDTA) at 4°C. To remove contaminating RNA, 250 mg of ribonuclease (RNase) was added per ml of dialysate and the solution incubated for 1 hour at 37°C. To remove the RNase, the

MS-1308

solution was extracted with an equal volume of phenol saturated with 0.1M Tris, pH 8, buffer. The upper phase from the phenol extraction was made 0.3M in sodium citrate and precipitated with 2 volumes of cold ethanol at -70°C for 30 minutes. The phage DNA was dissolved in TE buffer.

The M13-10 replicative form DNA was then cleaved by a double digest with Sal I, EcoRI under appropriate conditions such that the 800 bp (base pair) insert of the tuf A gene fragment was cut out of the vector.

E.    Isolation and radioactive labeling of the tuf A fragment

The double digested Sal I, EcoRI M13-10 DNA was adjusted to a concentration of 40 μg per 100 μl. Horizontal agarose gel electrophoresis was performed in a Bio-Rad Sub-Cell$^{TM}$ electrophoresis chamber (available from Bio-Rad Laboratories), A 1 percent low gelling temperature (LGT) agarose gel (available from Miles Research Products, Naperville, IL) in 0.089M Tris borate, 0.089M boric acid, 0.002M EDTA (TBE), pH 8, buffer containing 0.5 μg/ml ethidium bromide was formed in the Sub-Cell apparatus. The M13-10 double digested DNA solution was loaded on this gel at a concentration of 7 μg per well. Electrophoresis was performed at 50 volts for 3 hours at ambient temperature. The insert tuf A fragment DNA was cut out of the gel. These gel pieces were placed in a 50 ml polypropylene tube and heated to 70°C until the gel melted. The molten gel was adjusted to a total volume of 10 ml with 0.2M NaCl, 0.02M Tris, pH 7.0, 0.001M EDTA. The DNA was isolated from this gel solution through the use of the Elutip$^{TM}$d columns commercially available from Schleicher & Schuell, Keene, NH.

MS-1308

The procedure used was that recommended by the supplier. The Elutip$^{TM}$d column was washed with 2 ml of a solution of 1.0M NaCl, 0.02M Tris-HCl, pH 7.3, 0.001M EDTA. The column was washed with 0.5 ml of a solution comprised of 0.2M NaCl, 0.02M Tris-HCl, pH 6.3, 0.001M EDTA. A 0.45 µm cellulose acetate disposable filter was attached to the syringe (available from Schleicher & Schuell) and the DNA sample loaded onto the column. The DNA adsorbs to the matrix of the column. The column was washed with 3 ml of a solution containing 0.2M NaCl, 0.02M Tris-HCl, pH 7.3, 0.001M EDTA. Elution of the DNA sample from the column was accomplished by removing the cellulose acetate filter and adding 0.4 ml of the solution containing 1M NaCl, 0.02M Tris-HCl, pH 6.3, 0.001M EDTA. The DNA was precipitated with cold ethanol and dissolved in 40 µl of water.

The tuf A gene fragment was labeled with $^{32}$P by nick translation as described below. A reaction mixture was prepared consisting of 0.94 µg of tuf A DNA fragment, 20 micromoles each of deoxycytidine triphosphate (dCTP), deoxyguanosine triphosphate (dGTP) and deoxythymidine triphosphate (dTTP), 0.05 Tris-HCl, pH 7.8, 0.005M magnesium chloride, 0.01M 2-mercaptoethanol, 0.50 µg of nuclease free bovine serum albumin (BSA), 2 units of DNA polymerase I (available from BRL, Gaithersburg, MD) 200 picograms DNase I, 0.0005M magnesium acetate, 250 µg glycerol, and 4.88 µmoles of $[\alpha^{32}P]$ deoxyadenosine triphosphate (dATP) with a specific activity of 410 Ci/mmol. The total reaction volume was adjusted to 50 microliters with distilled water and incubated at 15°C for one hour. The reaction was stopped by the addition of 5 microliters of 0.3M Na$_2$EDTA (pH 8.0) and extracted with an equal volume of Tris-saturated phenol. The extracted mixture was chromatographed on a Sephadex G-50 column (Pharmacia Fine Chemicals, Piscataway, NJ) to remove the unreacted deoxytriphosphates. The $^{32}$P-labeled DNA fragments were contained in a total volume of 400 microliters with a specific activity of 8 x 10$^7$ cpm per microgram of DNA.

F.    Hybridization protocol

The nitrocellulose membrane described in part B of this example was pretreated before hybridization by placing the membrane in a heat sealable bag (commercially available as Boil-A-Meal$^{TM}$ bags from Scars, Roebuck & Co., Chicago, IL) and adding 10 ml of a solution consisting of 50% formamide (Matheson, Coleman & Bell), 5XSSPE (1XSSPE = 0.18M NaCl, 0.01M sodium phosphate, 0.001M Na$_2$ EDTA, pH 7.0), 5XBFP (1XBFP = 0.02% w/v of bovine serum albumin, Ficoll $/\overline{MW}$. 400,00$\underline{0}/$ and polyvinyl pyrrolidone), 1% glycine, and 100 μg/ml of denatured, sonicated carrier salmon sperm DNA. The membrane was incubated in this solution for one hour at 42°C. The prehybridization solution was then removed. Four milliliters of a solution containing 50% v/v formamide, 5XSSPE, 1XBFP, 100 μg/ml sonicated salmon sperm DNA, 10% w/v sodium dextran sulfate 500 and 0.3% SDS was prepared. Half of this solution (2 ml) was added to the bag containing the membrane and mixed. The labeled probe DNA was denatured by heating the DNA in a solution of 10mM Tris (pH 7.5), 1mM EDTA for 10 minutes at 95°C and cooling it quickly in an ice bath. The denatured probe DNA was added to the remaining 2 ml of solution at a quantity sufficient to provide 5 x 10$^6$ cpm per membrane. The 2 ml of solution containing the probe was then added to the bag and the contents were mixed. The bag was sealed by using a heat-sealer.

The hybridization mixture was incubated for 20 hours at 42°C. The nitrocellulose membrane was removed from the hybridization solution and washed in a solution

MS-1308

of 2XSSPE and 0.2% SDS. This wash was repeated four times at 45°C. The membranes were air dried and placed in contact with X-ray film (Dupont Cronex 4) and placed in a film cassette fitted with a "Lightning Plus" intensifying screen (available from Dupont). The film was exposed to the X-ray film for an appropriate time (this varies depending on the specificity and specific activity of the probe DNA and rate of the hybridization reaction).

G.    Detection of the presence of a bacteria that had been isolated from urine of a human patient with a urinary tract infection.

In a typical experiment as in this example, the presence of $1X10^6$ bacteria comprised of Escherichia coli No. 063 was detected using the tuf A gene fragment as a probe. The presence of a Gram positive organism Bacillus subtilis, a non-urinary tract isolate, was not detected even when as many as $1X10^8$ cells were present.

                    EXAMPLE 2

A.    Isolation and purification of DNA from bacterial strains that have been isolated from patients with urinary tract infections.

When the test organism is of the family Enterobacteriaceae, genus and species Escherichia coli, Proteus mirabilis, Enterobacter cloacae, Enterobacter aerogenes, Citrobacter freundii, Hafnia alvei, Klebsiella pneumoniae, Klebsiella oxytoca, Morganella morganni, Serratia rubideae, Providencia stuartii, and Klebsiella ozaenae, and the Families Streptococcaceae, Enterococcus, Micrococcaceae, and Staphylococcus aureus, the procedure for the isolation of genomic DNA is

MS-1308

essentially the same with the exception of *Staphylococcus aureus.*

The general procedure for isolation of genomic DNA will be described for all the organisms named in the preceeding paragraph. Each of the aforesaid organisms was grown in 1 liter of TBB+CAA medium which is comprised of 1% tryptose, 0.3% beef extract, 0.1% casamino acids (available from Difco Laboratories) and 0.5% sodium chloride for 18 hours with aeration. Cells were harvested by centrifugation and washed in 75 ml of a solution of 0.15M NaCl and 0.015M trisodium citrate (1XSSC). The cells were suspended in a mixture of 25 ml of 1XSSC and 1.5 ml of 0.25M EDTA. Lysis and RNA degradation was accomplished by the addition of 12.5 mg of lysozyme (egg white crystalline lysozyme, commercially available from Miles Laboratories, Naperville, IL) and 7.5 mg RNAse followed by incubation for 1 hour at 37°C. A 0.7 ml volume of 25% SDS was added to the mixture and incubation was continued for 1 hour followed by the addition of 10 ml of distilled water and 5 ml of 5M sodium perchlorate. Protein was removed by extracting the lysate with 50 ml of chloroform-isoamyl alcohol in a ratio of 24:1. The upper phase was removed, 10 mg of pronase added, and the mixture incubated for 2 hours at 37°C with gentle agitation. This mixture was extracted with 50 ml of chloroform-isoamyl alcohol The aqueous phase was extracted once more with chloroform-isoamyl alcohol. The DNA in the upper phase was precipitated with two volumes of cold 95% ethanol. The DNA was dissolved in sterile distilled water or 0.1 XSSC.

In the case of *Staphylococcus aureus*, lysis was accomplished by using Lysostaphin (available commercially from Sigma Chemical Co., St. Louis, MO), adding 375 µg of this enzyme to lyse biomass recovered from 1 liter of culture.

MS-1308

The DNA concentrations were determined using the fluorescence assay as described by A.R.Morgan et al, Nucleic Acids Research 7:547-594(1979).

B       Immobilization of genomic DNA isolated from organisms onto a solid support.

Samples of the DNA from each of the organisms named in Part A of this example and DNA from Bacillus subtilis and human placenta (human placental DNA is commercially available from Sigma Chemical Co., St. Louis, MO) were adjusted to DNA concentrations of 0.2 $\mu g/\mu l$, 0.02 $\mu g/\mu l$, 0.002 $\mu g/\mu l$, 0.020 $ng/\mu l$, 0.02 $ng/\mu l$ and 2.0 $pg/\mu l$. These six DNA concentrations were applied to a solid support consisting of a Gene-Screen$^{TM}$ membrane (New England Nuclear, Boston, MA). The application of a precise volume of DNA solution to a constant surface area of the membrane was accomplished by the use of the Minifold$^{TM}$ filtration apparatus from Schleicher & Schuell, Inc., (see part B example 1). The Minifold apparatus was fitted with one 4" x 5 1/4" sheet of nitrocellulose membrane that had been prewetted in 1XSSC. Vacuum was applied to the Minifold apparatus and 5 microliters of each concentration of DNA from each of the organisms named above was added. After the membrane was air dried, the membrane was immersed in a denaturing solution consisting of 2.5M NaCl and 0.5M NaOH for 2 minutes. Subsequently, the membrane was immersed in neutralizing solution which consisted of 3M sodium acetate (pH 5.5). Excess solution was blotted from membrane with Whatman 3MM paper. The membrane was baked at 80°C in a vacuum oven for 1 hour.

MS-1308

C. Isolation and purification of M13-10 replicative form DNA.

See part D, Example 1.

D. Isolation and radioactive labeling of the tuf A gene fragment.

See part E, Example 1.

E. Hybridization protocol.

See part F, Example 1.

F. Detection of the DNA of bacteria that comprise ninety-five percent of urinary tract infections in humans.

In a typical experiment as in this example, the results were as shown in Table A.

MS-1308

## Table A

| Family | Genomic DNA-Organism | # of Strains | Amount of DNA Detected |
|---|---|---|---|
| *Enterobacteriaceae* | *Escherichia coli* | 6 | 1.0 nanogram |
| " | *Proteus mirabilis* | 2 | 10.0 " |
| " | *Enterobacter cloacae* | 2 | 10.0 " |
| " | *Enterobacter aerogenes* | 1 | 1.0 " |
| " | *Citrobacter freundii* | 1 | 1.0 " |
| " | *Hafnia alvei* | 1 | 1.0 " |
| " | *Klebsiella pneumoniae* | 5 | 10.0 " |
| " | *Klebsiella oxytoca* | 1 | 1.0 " |
| " | *Morganella morganii* | 2 | 10.0 " |
| " | *Serratia rubideae* | 1 | 10.0 " |
| " | *Providencia stuartii* | 2 | 10.0 " |
| " | *Klebsiella ozaenae* | 1 | 10.0 " |
| *Micrococcaceae* | *Staphylococcus aureus* | 1 | * |
| *Pseudomonadaceae* | *Pseudomonas aeruginosa* | 2 | 1.0 " |
| *Streptococcaceae* | *Enterococcus* | 1 | 100 0 " |
| *Bacillaceae* | *Bacillus subtilis* | 1 | * |
| | Human placental DNA | - | * |

\* = not detectable

The results indicate that the *tuf A* gene probe is able to hybridize with essentially all of the organisms that are etiologic agents of urinary tract infections. The control DNA samples, *Bacillis subtilis*, a soil microorganism, and human DNA does not hybridize with the *tuf A* gene.

## EXAMPLE 3

A. *Hybridization of DNA from urinary tract isolates lysed in situ on a nitrocellulose membrane filter.*

The following microorganisms were grown on TBAB (Tryptose Blood Agar Base) - Difco agar plates + casamino acids (CA) overnight to obtain single colony isolates:

> *Escherichia coli* A5
> *Escherichia coli* A6
> *Klebsiella pneumoniae* B4
> *Klebsiella pneumoniae* B5
> *Proteus mirabilis* C2
> *Enterobacter cloacae* D4
> *Morganella morganii* C7

A single colony isolate from each type of microorganism was used as inoculum for ten ml of TBAB + CA broth. These cultures were grown overnight at 37°C. Each culture was diluted $10^{-2}$ to $10^{-7}$ in TBAB + CA media at 4°C to stop all growth. Each dilution was plated on TBAB + CA agar plates to determine the number of viable organisms per ml of media. Also, one ml of each dilution was transferred to a siliconized tube. To each tube was added: 50 microliters lysozyme (10 mg/ml) dissolved in 10 mM Tris, 1 mM EDTA buffer, pH 8, 100 microliters of 1% SDS dissolved in 0.2 N NaOH (sodium hydroxide), and 0.5 ml modified 20x SSPE which consists of 3.6 M NaCl,

MS-1308

0.2 M $NaH_2PO_4 \cdot H_2O$, 0.16 M NaOH and 0.020 M $Na_2EDTA$. The SSPE is modified by adding sufficient 0.2 N NaOH solution to adjust the pH of the 20x SSPE to 7.4. The purpose of this mixture is to disrupt and lyse the bacterial cells and denature the DNA that is released from the cells.

The entire lysed cell mixture samples from each cell type were applied to a solid nitrocellulose support following the method of part B, Example 1. To each well of the Minifold apparatus was added the entire volume of cell lysate (which contains 1 ml of broth containing the cells and dilutions plus lysis reagents) of each sample.

A control culture of mammalian cells at a concentration of $1 \times 10^7$ cells per ml (determined by hemocytometer count) and serial dilutions of these cells was also lysed and collected on the filter in the same manner as described for the cultures of microorganisms. Each sample and control was done in duplicate.

The nitrocellulose membrane described above in this example was pretreated before hybridization by placing the membrane in the heat sealable bag commercially available from Sears Roebuck & Co., and adding 10 ml of a solution consisting of 5XBFP (1XBFP = 0.02% w/v bovine serum albumin, Ficoll of M.W. 400,000, and polyvinylpyrrolidone), 5XSSPE, 0.3% SDS, and 100 µg/ml salmon sperm DNA. The membrane was incubated in this solution for one hour at 70°C and the prehybridization solution removed. Ten milliliters of a solution containing 5XSSPE, 1XBFP, 100 µg/ml salmon sperm DNA and 0.3% SDS was prepared. Half of this solution (5 ml) was added to the bag containing the membrane and mixed. The labeled probe DNA (*tuf* fragment from part E, Example 1) was denatured by heating the DNA in a solution of 10 mM Tris (pH 7.5),

0133288

1 mM EDTA for 10 min. at 95°C and cooling it quickly in an ice bath. The denatured probe DNA was added to the remaining 5 ml of solution at a quantity sufficient to provide $5 \times 10^6$ cpm per membrane. The solution containing the probe was added to the bag and the bag was heat sealed.

The hybridization mixture was incubated for 16 hours at 70°C. The nitrocellulose membrane was removed from the hybridization solution and washed in a solution of 5XSSPE and 0.5% SDS. This wash was repeated four times at 70°C. The membranes were air dried; subsequently, the membranes were cut up in such a manner as to cut out the filter area encompassed by each well of the Minifold as well as an equivalent border area for each well. These filter squares were placed into individual scintillation vials containing 5 ml of Aquasol$^{TM}$ fluor (New England Nuclear, Villerica, MA). Duplicate samples of each cell type and its' serial dilutions were counted on a Beckman scintillation counter.

The results of this experiment are reported in Table B.

MS-1308

## Table B

| Cell Type | Number of Viable Cells Applied to Each Well of Minifold™ | ³²P-CPM-x Counts Per Minute-Measure of Extent of Hybridization of *tuf* Gene to DNA of Organism | Standard Deviation - CPM |
|---|---|---|---|
| *Escherichia coli* #057 | $1.5 \times 10^8$ | 2,339 | 235 |
|  | $1.5 \times 10^7$ | 699 | 122 |
|  | $1.5 \times 10^6$ | 648 | 64 |
|  | $1.5 \times 10^5$ | 114 | 11 |
| *Escherichia coli* #163 | $1.7 \times 10^8$ | 4,481 | 676 |
|  | $1.7 \times 10^7$ | 1,262 | 304 |
|  | $1.7 \times 10^6$ | 676 | 106 |
|  | $1.7 \times 10^5$ | 118 | 14 |
| *Klebsiella pneumoniae* | $1.1 \times 10^8$ | 3,062 | 586 |
|  | $1.1 \times 10^7$ | 670 | 111 |
|  | $1.1 \times 10^6$ | 494 | 114 |
|  | $1.1 \times 10^5$ | 102 | 9 |
| *Klebsiella pneumoniae* | $1.0 \times 10^8$ | 1,809 | 160 |
|  | $1.0 \times 10^7$ | 430 | 69 |
|  | $1.0 \times 10^6$ | 111 | 12 |
|  | $1.0 \times 10^5$ | 98 | 5 |
| *Proteus mirabilis* | $2.0 \times 10^8$ | 2,677 | 653 |
|  | $2.0 \times 10^7$ | 699 | 122 |
|  | $2.0 \times 10^6$ | 647 | 64 |
|  | $2.0 \times 10^5$ | 114 | 11 |
| *Enterobacter cloacae* | $5.0 \times 10^7$ | 1,341 | 71 |
|  | $5.0 \times 10^6$ | 315 | 15 |
|  | $5.0 \times 10^5$ | 194 | 26 |
| *Morganella morganii* | $1.0 \times 10^8$ | 1,251 | 267 |
|  | $1.0 \times 10^7$ | 217 | 32 |
|  | $1.0 \times 10^6$ | 161 | 7 |
| Mammalian Cells | $1.0 \times 10^7$ | 90 | 28 |
|  | $1.0 \times 10^6$ | 69 | 8 |

- 42 -

The present invention has been particularly described and exemplified above. Obviously, many other variations and modifications of the invention may be made without departing from the spirit and scope hereof.

MS-1308

0133288

CLAIMS:

1. A method for detecting bacteria in a test sample, comprising the steps of subjecting the test sample to conditions to release and denature nucleic acids from bacteria present in the sample, contacting the resulting single stranded nucleic acids with a polynucleotide probe under conditions favorable to hybridization between said probe and bacterial base sequences, and determining the presence of hybridized probe, characterized in that the probe comprises a base sequence of at least a portion of one of the strands of a gene selected from tuf and fus genes.

2. The method of Claim 1 wherein said gene is an E. coli tuf gene.

3. The method of Claim 1, characterized in that it comprises the steps of:

(a) immobilizing the single stranded nucleic acids resulting from treatment of the test sample on a solid support,

(b) contacting the solid support with a labeled polynucleotide probe having a base sequence of at least a 10 base portion of one of the strands of a tuf gene sequence,

(c) separating unhybridized labeled probe from said solid support, and

(d) determining hybridized labeled probe associated with said support by measuring the label comprised in said labeled probe.

4. The method of Claim 3, characterized in that step (b) involves adsorbing the single stranded DNA resulting from step (a) onto said solid support and thereafter

MS-1308

treating the support with foreign nucleic acid to substantially saturate all remaining nucleic acid adsorption sites thereon.

5.    A labeled polynucleotide probe suitable for the method of Claim 1, characterized in that it comprises a label incorporated with a polynucleotide probe comprising a base sequence of at least a portion of one of the strands of a gene selected from tuf and fus genes.

6.    A labeled probe according to Claim 5, characterized in that said homologous base sequence is at least about 10 bases in length.

7.    A labeled probe according to Claim 5 or 6, characterized in that said gene is an E. coli tuf gene.

8.    A test kit suitable for the method of Claim 1, characterized in that it comprises:

(a)    a solid support capable of immobilizing single stranded nucleic acid resulting from treatment of said test sample to release and denature genomic nucleic acids from bacteria present in said sample, and

(2)    a labeled polynucleotide probe comprising a base sequence of at least a portion of one of the strands of a gene selected from tuf and fus genes.

9.    A test kit according to Claim 8, characterized in that it additionally comprises foreign nucleic acid for use in substantially saturating nucleic acid adsorption sites on said support after immobilization of said single stranded nucleic acids from bacteria present in said sample.

MS-1308

0133288

10. A test kit according to Claim 8 or 9, characterized in that it additionally comprises a chemical lysing and denaturation agent for treating said test sample to release single stranded genomic nucleic acids from bacteria present therein.